# EUROPÄISCHE PATENTSCHRIFT

(11) **EP 3 407 890 B1**
(45) Veröffentlichungstag und Bekanntmachung des Hinweises auf die Patenterteilung: **19.07.2023**
(21) Anmeldenummer: 17700818.2
(22) Anmeldetag: 18.01.2017
(51) Int. Cl.: A61P 31/12, A61K 45/06, A61K 38/38, A61K 36/534, A61K 33/38, A61K 31/728, A61K 31/7024, A61K 31/58, A61K 31/573, A61K 31/155, A61K 9/12

(54) **PHARMAZEUTISCHE ZUBEREITUNG UND DEREN VERWENDUNG IN DER BEHANDLUNG VON VIRALER LARYNGITIS**
PHARMACEUTICAL PREPARATION AND THE USE THEREOF FOR TREATING VIRAL LARYNGITIS
PRÉPARATION PHARMACEUTIQUE ET SON UTILISATION POUR LE TRAITEMENT DE LA LARYNGITE VIRALE

(30) Priorität: 27.01.2016 DE 202016100357 U
(43) Veröffentlichungstag der Anmeldung: 05.12.2018
(73) Patentinhaber: Sommer, Peter, 40212 Düsseldorf (DE); Vaubel, Nicola, 40545 Düsseldorf (DE)
(72) Erfinder: Sommer, Peter, 40212 Düsseldorf (DE); Vaubel, Nicola, 40545 Düsseldorf (DE)
(74) Vertreter: Cohausz & Florack
(86) Internationale Anmeldenummer: PCT/EP2017/050938
(87) Internationale Veröffentlichungsnummer: WO 2017/129457

(56) Entgegenhaltungen:
- EP-A1- 0 579 435
- EP-A1- 1 595 537
- EP-A1- 2 110 116
- EP-A1- 2 614 812
- WO-A1-92/18133
- WO-A1-2014/145602
- WO-A2-2009/106963
- US-A1- 2010 190 735
- J. M. WOOD ET AL: "Laryngitis", BMJ, vol. 349, no. oct09 21, 9 October 2014 (2014-10-09), pages g5827-g5827, XP055686402, DOI: 10.1136/bmj.g5827

## Beschreibung

Die Erfindung betrifft eine pharmazeutische Zubereitung zur topisch-oralen Anwendung auf Schleimhäuten im Kehlkopf- und/oder Rachenraum und deren Verwendung zur symptomatischen Therapie einer viralen Laryngitis.

Die pharmazeutische Zubereitung kann erfolgreich zur Vorbeugung des Auftretens von Symptomen, zur Symptomlinderung und zur ursächlichen Therapie entzündlicher, virusbedingter Laryngitis eingesetzt werden.

Atemwegserkrankungen sind Krankheiten, die sich primär am Atmungsapparat, insbesondere an den Schleimhäuten des Atmungsapparats, abspielen. Atemwegserkrankungen werden nach ICD 10 ("International Classification of Diseases") eingeteilt. Besonders entzündliche Infektionen der oberen Atemwege (ICD 10, J00-J06), beispielsweise Pharyngitis, Tonsillitis, Laryngitis, sind derzeit häufig nur symptomatisch therapierbar. Dies gilt vor allem für virale, entzündliche Erkrankungen der oberen Atemwege.

Die EP 2 110 116 A1 beschreibt eine wässrige antiseptische Zubereitung zur Behandlung des Mund- und Rachenraums, die neben Chlorhexidin pflanzliche Wirkstoffe und ein Alkali- und/oder Erdalkalifluorid enthält. Diese Zubereitung dient der Desinfektion und Entzündungshemmung im Mund- und Rachenraum. Die EP 1 595 537 A1 beschreibt eine antimikrobielle Zubereitung zur Behandlung der Mundhöhle, die eine aus Chlorhexidin und Triclosan zusammengesetzte Verbindung mit einem Zinksalz enthält. Durch diese Zusammensetzung soll der Entfärbung von Zähnen durch Chlorhexidin entgegengewirkt werden. Die WO 2009/106963 A2 beschreibt eine dentale Zubereitung zur Vorbeugung und Behandlung von Stomatitis, Ulzera der Mundschleimhaut und Schädigungen der mukösen Membran. Eingesetzt wird dazu ein flüssiger Myrrhe-Extrakt und ein lösliches Zinksalz neben fakultativ einsetzbaren Verbindungen wie Chlorhexidin-Digluconat. Die EP 2 614 812 A1 beschreibt eine Mundspülung, die Chlorhexidin, ein Alkali- oder Erdalkalimetallmetabisulfitsalz, Ascorbinsäure und Hyaluronsäure in Salz- oder Komplexform enthält. Die WO 92/18133 A1 beschreibt die Behandlung von entzündlichen Erkrankungen des Mundes unter Verwendung eines Kortikoids, eines Antihistamins, eines lokalen Anästhetikums und eines antibiotisch wirksamen Fungizids in einem wässrigen Medium als Mundspülung. Die US 2010/0190735 A1 beschreibt eine Kortison enthaltende pharmazeutische Zubereitung für die Mundhygiene, insbesondere Mundspülungen zur Behandlung und Vorbeugung von Mukositis und Stomatitis. Die EP 0 579 435 A1 beschreibt die Verwendung von bestimmten Polymeren bei der Herstellung von Cyclodextrin-Arzneistoff-Komplexen zur Erhöhung der Löslichkeit und Stabilität der Cyclodextrinderivate von Arzneistoffen, die damit komplexiert sind. Die WO 2014/145602 A1 beschreibt eine Zusammensetzung, die ein Polyphenol und einen im Mundraum verträglichen Träger umfasst, wobei das Polyphenol in Form eines Alkalimetallsalzes oder eines Konzentrats vorliegt. Laryngitis, BMJ 2014;349:g5827 liefert eine Übersicht zur Laryngitis und schlägt zur Therapie Stimmhygiene und Erythromycin vor.

Als Laryngitis wird die Entzündung der Kehlkopfschleimhaut bezeichnet, die zur Heiserkeit bis hin zur Stimmlosigkeit führt und sehr häufig mit einem trockenen, quälenden Husten einhergeht. Weitere Symptome sind starke Halsschmerzen oder Fieber. Die Laryngitis kann sowohl in akuter als auch chronischer Form auftreten. Die Unterteilung der akuten Form erfolgt in eine Entzündung der verschiedenen Etagen des Larynx, der Supraglottis, Glottis, Subglottis. Die akute Laryngitis wird überwiegend durch Virusinfekte, beispielsweise durch den Rhinovirus, Influenzavirus, Parainfluenzavirus, Adenovirus, Coxsackievirus, Coronavirus und respiratorischen Synzytial-Virus, der oberen Atemwege oder durch starke Stimmbelastung verursacht. Nicht selten entwickelt sich durch die rein symptomatische Behandlung und/oder geringe Compliance des Patienten aus dem akuten Zustand eine chronische Erkrankung. Weiterhin tritt häufig anschließend oder parallel zu der akuten oder chronischen viralen entzündlichen Erkrankung der oberen Atemwege eine bakterielle Sekundärinfektion auf, die eine Antibiotikatherapie erfordert. Beim Einsatz von Antibiotika sind unterschiedlichste Aspekte zu beachten, die den Therapierfolg bestimmen, beispielsweise die Wahl des richtigen Antibiotikums, Resistenzbildung sowie Compliance des Patienten.

In der medizinischen Praxis hat sich oftmals gezeigt, dass virale Infektionen der oberen Atemwege schwer ursächlich zu therapieren sind. Bekannte synthetische, antivirale Wirkstoffe sind nicht für die Therapie viraler, entzündlicher Erkrankungen der oberen Atemwege, insbesondere der viralen Laryngitis, geeignet oder zugelassen. Die derzeitige Therapie bei viralen, entzündlichen Erkrankungen der oberen Atemwege, insbesondere bei der viralen Laryngitis, besteht in einer rein symptomatischen Behandlung. So besteht die Therapie vordergründig aus Stimmschonung, Inhalation zur Befeuchtung der Schleimhäute, Hustenblockern zur Linderung des Reizhustens, Schleimlösern, sowie schmerzstillenden und entzündungshemmenden Medikamenten, die lokal oder systemisch angewendet werden können. Es müssen daher parallel unterschiedliche Medikamente zu unterschiedlichen Tageszeiten von dem Patienten mehrfach eingenommen bzw. angewendet werden.

Nachteilig an den bisher bekannten Behandlungsmethoden ist, dass nur eine unvollständige Linderung der Symptome und keine Bekämpfung der viralen Ursache möglich ist, die häufig zu einer sich daran anschließenden bakteriellen Sekundärinfektion führt. Weiterhin müssen mehrere Präparate gleichzeitig und regelmäßig über den ganzen Tag angewendet werden, was zu einer verminderten Compliance des Patienten führen und dadurch den Therapieerfolg gefährden kann. Durch eine unzureichende Therapie kann sich so aus einer anfänglich akuten eine manifeste, chronische Entzündung der oberen Atemwege mit einhergehender bakterieller Sekundärinfektion entwickeln. Derzeit befindet sich kein Kombinationspräparat auf dem Markt, das die Symptome lindert und die virale Grunderkrankung therapiert.

Ausgehend von dem vorstehend erläutertem Stand der Technik und dessen Nachteilen, besteht eine Aufgabe der Erfindung darin, eine alternative antivirale pharmazeutische Zubereitung zur symptomatischen Therapie viral bedingter, entzündlichen Erkrankungen der oberen Atemwege bereitzustellen.

Wenn hier oder an anderer Stelle von viral bedingten, entzündlichen Erkrankungen der oberen Atemwege die Rede ist, so ist damit die virale Laryngitis gemeint, die zu den viralen, entzündlichen Erkrankungen der oberen Atemwege gemäß ICD 10, bevorzugt viral bedingten, entzündlichen Erkrankungen des Mund-/Rachenraums (J00-J06) gehört.

Diese Aufgabe wird erfindungsgemäß gelöst durch eine pharmazeutische Zubereitung zur topisch-oralen Anwendung auf Schleimhäuten im Rachen- und Kehlkopfraum, umfassend mindestens die folgenden Komponenten
a) 0,01 bis 0,7 Gew.-% eines Kortikoids,
b) 0,01 bis 5 Gew.-% einer Metallverbindung,
c) 0,01 bis 5 Gew.-% eines Adstringens und
d) 0,005 bis 0,03 Gew.-% eines Desinfektionsmittels,
e) 0,01 bis 0,06 Gew.-% Hyaluronsäure/Hyaluronsäuresalz,
worin die Zubereitung mit einem oder mehreren pharmazeutisch verträglichen Lösungsmittel/n zu 100 Gew.-% ergänzt wird, zur symptomatischen Behandlung der viralen Laryngitis.

Obwohl die einzelnen Komponenten für sich genommen eine Vielzahl von Wirkungen aufweisen können, vermögen die Komponenten einzeln angewendet nur eine Linderung der Symptome zu bewirken. Überraschenderweise hat sich gezeigt, dass die erfindungsgemäße pharmazeutische Zubereitung nicht nur die Symptome viraler, entzündlicher Erkrankungen der oberen Atemwege lindert, sondern auch antivirale Wirkung entfaltet. Damit kann auch die häufig auftretende bakterielle Sekundärinfektion präventiv therapiert werden.

In praktischen Versuchen hat sich zudem gezeigt, dass erst die Kombination der oben genannten Komponenten zu einer erfolgreichen Therapie entzündlicher, virusbedingter Erkrankungen der oberen Atemwege führt. Als besonders effektiv hat sich die erfindungsgemäße pharmazeutische Zubereitung zur Therapie der viralen Laryngitis erwiesen. Wenn hier oder im Folgenden von Laryngitis die Rede ist, dann sind damit alle Formen der viralen Laryngitis gemeint.

Durch die Anwendung der erfindungsgemäßen pharmazeutischen Zubereitung können virale, entzündliche Erkrankungen der oberen Atemwege erfolgreich therapiert werden, das Risiko einer bakteriellen Sekundärinfektion kann vermindert oder sogar ihre Entstehung gänzlich verhindert werden. Daher kann die erfindungsgemäße pharmazeutische Zubereitung präventiv, im akuten oder chronischen Krankheitsstadium eingesetzt werden.

Vorteilhaft ist ferner, dass die erfindungsgemäßen pharmazeutische Zubereitung, die mindestens die Komponenten a) bis e) enthält, ein Kombinationspräparat ist und nicht wie bisher mehrere unterschiedliche Medikamente in verschieden Applikationsformen angewendet werden müssen, die wiederum zu unterschiedlichen Zeitpunkten eingenommen werden müssen. Damit kann nicht nur Anzahl der einzunehmenden Medikamente deutlich gesenkt werden, sondern auch die Compliance des Patienten und damit einhergehend der Therapieerfolg gesteigert werden. Dies kann zudem zur Senkung der Gefahr der Entwicklung einer chronischen, entzündlichen Atemwegserkrankung sowie zur Senkung der Kosten der täglich anzuwendenden Dosis beitragen.

Ohne an eine bestimmte Theorie gebunden zu sein, wird die antientzündliche und antivirale Wirkung wahrscheinlich durch die synergistische Wirkung der Komponenten a) bis e) entfaltet. Es hat sich gezeigt, dass die erfindungsgemäße pharmazeutische Zubereitung auch Wundinfektionen verhindern, entzündliche Prozesse auf den Schleimhäuten eindämmen oder beseitigen und antiviral wirken kann. Wenn hier oder anderer Stelle von "antiviral" die Rede ist, dann ist damit gemeint, dass die Viruslast gesenkt, der Virus inaktiviert, zerstört, das virale Wachstum, die Vermehrung und/oder Ausbreitung gehemmt wird.

Vorteilhaft ist weiterhin, dass durch die sich gegenseitig unterstützende oder verstärkende Wirkung der Einzelkomponenten insgesamt die Dosis der einzelnen Komponenten gesenkt werden kann und der Körper dadurch eine geringere toxikologische Belastung erfährt.

In praktischen Versuchen hat sich weiterhin gezeigt, dass die Komponenten auf einfache Weise kombiniert und die erfindungsgemäße pharmazeutische Zubereitung ohne gro-βen Herstellungsaufwand erhalten und kostengünstig hergestellt werden kann.

Ein weiterer Vorteil ist, dass die erfindungsgemäße pharmazeutische Zubereitung derart galenisch formuliert werden kann, sodass sie in einer für den Patienten einfach anwendbaren und pharmazeutisch verträglichen Applikationsform vorliegt.

Wenn hier oder anderer Stelle von "pharmazeutisch verträglich" die Rede ist, dann ist damit gemeint, dass die jeweilige Komponente oder Mischungen der Komponenten zur Verwendung in einer pharmazeutischen Zubereitung geeignet und/oder für Mensch oder Tier unbedenklich ist.

Die erfindungsgemäße pharmazeutische Zubereitung enthält als Komponente a) ein Kortikoid. Die Wirkstoffklasse der Kortikoide, die pharmakologische Wirkung, die Auswahl des pharmazeutisch anzuwendenden Kortikoids, die Anwendungsgebiete bzw. Indikationen und Dosierungen sind dem Fachmann allgemein bekannt (vgl. Arzneimittelwirkungen , Pharmakologie - Klinische Pharmakologie - Toxikologie, Ernst Mutschler, Gerd Geisslinger, Heyo K. Kroemer, Sabine Menzel, Peter Ruth, 2001, S. 721ff). Die erfindungsgemäße pharmazeutische Zubereitung enthält die Komponente a)in einer Menge von 0,01 bis 0,7 Gew.-%, bevorzugt von 0,045 bis 0,075 Gew.-% und besonders bevorzugt von 0,05 bis 0,065 Gew.-%, bezogen auf das Gesamtgewicht der pharmazeutischen Zubereitung. Als Kortikoid im Sinne der Erfindung kann Dexamethason, Hydrocortison, Prednisolon, Clobetason, Flumetason, Fluocortin, Fluperolon, Fluorometholon, Flupredniden, Desonid, Triamcinolon, Alclometason, Hydrocortisonbuteprat, Clocortolon, Betamethason, Fluclorolon, Desoximetason, Fluocinolonacetonid, Fluocortolon, Diflucortolon, Fludroxycortid, Fluocinonid, Budesonid ,Diflorason, Amcinonid, Halometason, Methylprednisolonaceponat, Beclometason, Hydrocortisonaceponat, Fluticason, Prednicarbat, Difluprednat, Ulobetasol, Clobetasol, Halcinonid, deren pharmazeutisch verträgliche Salze und/oder Kombinationen davon eingesetzt werden.

Wenn hier oder anderer Stelle von "pharmazeutisch verträglichen Salzen" die Rede ist, dann ist damit gemeint, dass das Salz oder die Salzform der Komponente zur Verwendung in einer pharmazeutischen Zubereitung geeignet und/oder für Mensch oder Tier unbedenklich ist. Dem Fachmann sind derartige pharmazeutisch verträglichen Salze grundsätzlich bekannt.

Die erfindungsgemäße pharmazeutische Zubereitung enthält als Komponente b) ein Metall. Die erfindungsgemäße pharmazeutische Zubereitung enthält die Komponente b) in einer Menge von 0,01 bis 5 Gew.-%, bevorzugt von 0,03 bis 4 Gew.-% und besonders bevorzugt von 0,02 bis 3,5 Gew.-%, bezogen auf das Gesamtgewicht der pharmazeutischen Zubereitung. Das Metall kann ausgewählt sein aus Mn, Ag, Zn, Sn, Fe, Cu, Al, Ti und Kombinationen davon. Es sind aber auch weitere Metalle denkbar, die eine antivirale Wirkung entfalten können. Das Metall kann als pharmazeutisch verträgliches Salz, in einer pharmazeutisch verträglichen Form und/oder Zubereitungen davon vorliegen. In einer Ausführungsform der Erfindung kann das Metall als Pulver, Lösung, kolloidale Lösung oder Suspension vorliegen. In einer weiteren Ausführungsform kann das Metall in einer Metall-Eiweißverbindung oder einem Metall-Eiweißkomplex vorliegen. In einer Ausführungsform der Erfindung kann die Metall-Eiweißverbindung oder Metall-Eiweißkomplex als Salz, in einer Verbindung oder einem Komplex mit einer der Komponenten a), c), d), e) und/oder einem weiteren Zusatzstoff vorliegen. In einer bevorzugten Ausführungsform liegt das Metall in einer Silber-Eiweißverbindung vor. In einer besonders bevorzugten Ausführungsform liegt das Metall als Silbereiweiß, insbesondere Silbereiweißacetyltannat vor.

Die erfindungsgemäße pharmazeutische Zubereitung enthält als Komponente c) ein Adstingens. Adstingentia sind dem Fachmann allgemein bekannt (Pschyrembel klinisches Wörterbuch, Willibald Pschyrembel, Otto Dornblüth, Christoph Zink, S. 23, 1986). Die Menge des in der erfindungsgemäßen pharmazeutischen Zubereitung enthaltenden Komponente c) beträgt 0,01 bis 5 Gew.-%, bevorzugt von 0,03 bis 4 Gew. -% und besonders bevorzugt von 0,02 bis 3,5 Gew.-%, bezogen auf das Gesamtgewicht der pharmazeutischen Zubereitung. Das Adstringens kann ausgewählt sein aus Catechingerbstoffen, polymeren Proanthocyanidinen, oligomeren Proanthocyanidinen, Oligomeren oder Polymeren der Catechine, Catechin, Epicatechin, Flavonoiden, hydrolysierbaren Gerbstoffen, Gallotanninen, Estern der Gallussäure oder Derivaten davon, Tanninen, Kaffeesäure- und Phloroglucinderivaten, Labiatengerbstoffen, Rosmarinsäure oder Derivaten davon, Alaun, Tonerde und Kombinationen davon.

Weiterhin umfasst die erfindungsgemäße pharmazeutische Zubereitung als Komponente d) ein Desinfektionsmittel. Desinfektionsmittel zu pharmazeutischen Zwecken sind dem Fachmann allgemein bekannt (Pschyrembel klinisches Wörterbuch, Willibald Pschyrembel, Otto Dornblüth, Christoph Zink, S. 340, 1986). Die erfindungsgemäße Zubereitung enthält die Komponente d) in einer Menge von 0,005 bis 0,03 Gew.-%, bevorzugt von 0,005 bis 0,02 Gew.-% und besonders bevorzugt von 0,008 bis 0,01 Gew.-%, bezogen auf das Gesamtgewicht der pharmazeutischen Zubereitung. Das Desinfektionsmittel kann ausgewählt sein aus Chlorhexidin, Hexetidin, Ethanol, Propanol, Dichlorbenzylalkohol, Amylmetakresol, Menthol, Levomenthol, Dequaliniumchlorid, Iod, Povidon-Iod, Gentianaviolett und Kombinationen davon.

Die erfindungsgemäße Zubereitung enthält als Komponente e) Hyaluronsäure. Aufbau, Vorkommen, Wirkung sowie pharmazeutisch anwendbare Formen der Hyaluronsäure sind dem Fachmann allgemein bekannt (Pschyrembel klinisches Wörterbuch, Willibald Pschyrembel, Otto Dornblüth, Christoph Zink, S. 718, 1986). Die Menge der in der erfindungsgemäßen pharmazeutischen Zubereitung enthaltenden Komponente e) beträgt 0,01 bis 0,06 Gew.-%, bevorzugt von 0,02 bis 0,04 Gew.-% und besonders bevorzugt von 0,025 bis 0,035 Gew.-%, bezogen auf das Gesamtgewicht der pharmazeutischen Zubereitung. Die Hyaluronsäure kann als pharmazeutisch verträgliches Salz, in einer pharmazeutisch verträglichen Form und/oder Zubereitungen davon vorliegen. In einer Ausführungsform der Erfindung liegt die Hyaluronsäure als Natrium-Salz der Hyaluronsäure (Natriumhyaluronat) vor.

Die erfindungsgemäße pharmazeutische Zubereitung wird mindestens mit einem bzw. einem oder mehreren pharmazeutisch verträglichen Lösungsmittel/n zu 100 Gew.-%, bezogen auf das Gesamtgewicht der pharmazeutischen Zubereitung ergänzt. Das Lösungsmittel kann Wasser und/oder Ethanol sein. In einer Ausführungsform ist das Lösungsmittel Wasser, insbesondere gereinigtes Wasser (Aqua purificata). In einer besonders bevorzugten Ausführungsform enthält die pharmazeutische Zubereitung gereinigtes Wasser und Ethanol. Sie ist vorzugsweise sprühbar und weist vorzugsweise die folgende Zusammensetzung auf:

| | | | |
|---|---|---|---|
| Dexamethasonacetat | | 0,065 | Gew.-% |
| Silbereiweißacetyltannat | | 3,0 | Gew.-% |
| Chlorhexidingluconat | | 0,01 | Gew.-% |
| Hyaluronsäure-NaSalz | | 0,03 | Gew.-% |
| Ethanol (90%) | | 25,0 | Gew.-% |
| Pfefferminzöl | | 0,29 | Gew.-% |
| Rest Aqua purificata | ad | 100 | Gew.-%. |

Denkbar ist auch ein pulverbasiertes Spray.

Weiterhin kann die pharmazeutische Zubereitung mindestens eine weitere Komponente f) und/oder g) aufweisen. In einer Ausführungsform der Erfindung kann in der pharmazeutischen Zubereitung als zusätzliche Komponente f) mindestens ein ätherisches Öl in einer Menge von 0, 01 bis 0,4 Gew.-%, bevorzugt von 0,1 bis 0,35 Gew.-% und besonders bevorzugt von 0,2 bis 0,3 Gew.-%, bezogen auf das Gesamtgewicht der pharmazeutischen Zubereitung, enthalten sein. Das ätherische Öl kann aus Orange, Kamille, Schafgarbe, Salbei und/oder der Pfefferminze stammen oder daraus erhältlich sein. In einer bevorzugten Ausführungsform wird Pfefferminzöl (Oleum menthae piperitae) als ätherisches Öl in der pharmazeutischen Zubereitung eingesetzt. In einer anderen Ausführungsform enthält die pharmazeutische Zubereitung als zusätzliche Komponente g) Ethanol (90%) in einer Menge von 10,0 bis 40,0 Gew.-%, bevorzugt von 20 bis 30 Gew.-% und besonders bevorzugt von 23 bis 26 Gew.-%, bezogen auf das Gesamtgewicht der pharmazeutischen Zubereitung.

Optional kann die pharmazeutische Zubereitung neben den Komponenten a) bis e) zusätzlich mindestens einen weiteren Zusatzstoff enthalten. Der Zusatzstoff kann zur Angleichung des pH-Wertes, der Viskosität, der Verbesserung der Lösungseigenschaften der einzelnen Komponenten, Stabilität, Haltbarkeit, des Geschmacks oder Aussehens dienen. Der Zusatzstoff kann auch ein weiterer Stoff sein, der die Linderung der Symptome und/oder die antivirale Wirkung unterstützt. Dem Fachmann sind grundsätzlich derartige Zusatzstoffe bekannt. Zusatzstoffe im Sinne er Erfindung können ausgewählt sein aus pharmazeutischen Hilfsstoffen, galenischen Hilfsstoffen, Zuckeraustauschstoffen, Zuckerersatzstoffen, Säuerungsmitteln, Lösungsvermittlern, Verdickungsmitteln, Füllmitteln, Farbstoffen, Konservierungsmitteln, Aromen, Geschmacksstoffen, Lokalanästhetika, Provitaminen, insbesondere Dexpanthenol, Vitaminen und Kombinationen davon.

In einer besonders bevorzugten Ausführungsform liegt der pH-Wert der pharmazeutischen Zubereitung in einem physiologischen-verträglichen pH-Bereich. Typischerweise zeigt die pharmazeutische Zubereitung einen pH-Wert von 3,5 bis 7,5.

In einer bevorzugten Ausführungsform der Erfindung enthält die pharmazeutische Zubereitung mindestens eine zusätzliche Komponente f), g) und/oder einen weiteren Zusatzstoff.

In einer Ausführungsform der Erfindung liegt die pharmazeutische Zubereitung in einer geeigneten galenischen Formulierung vor. Wenn hier von "geeigneter galenischer Formulierung" die Rede ist, dann ist damit gemeint, dass die pharmazeutische Zubereitung in einer Form vorliegt, welche zur Anwendung im oberen Atemwegsbereich geeignet ist. In einer Ausführungsform der Erfindung kann die pharmazeutische Zubereitung direkt in die Atmungspassage oder auf Schleimhäute von Rachen- und/oder Mundraum appliziert werden. Galenischen Formulierungen im Sinne der Erfindung können Lösungen, kolloidale Lösungen, Suspensionen, Tropfen, Sprays, insbesondere Pumpsprays, Drucksprays, Zerstäuber, Luftinhalationsvorrichtungen und/oder bekannte geeignete Applikationsformen sein. In einer weiteren Ausführungsform enthält die pharmazeutische Zubereitung pharmazeutische und/oder galenische Hilfsstoffe, die zur Herstellung einer geeigneten galenischen Formulierung beitragen. In einer bevorzugten Ausführungsform liegt die pharmazeutische Zubereitung als Spray vor. Derartige Sprays können besonders einfach im Rachenraum angewendet werden. Sie sind geeignet, die pharmazeutische Zubereitung an schwer zugängliche Stellen im Mund-/Rachenbereich und/oder Kehlkopfbereich aufzubringen.

Die erfindungsgemäße Zubereitung kann auch auf die zu therapierenden Bereiche aufgepinselt werden.

Die folgenden Beispiele dienen der weiteren Erläuterung der Erfindung.

### Beispiel 1

Die folgenden Komponenten

| | | | |
|---|---|---|---|
| Dexamethasonacetat | | 0,065 | Gew.-% |
| Silbereiweißacetyltannat | | 3,0 | Gew.-% |
| Chlorhexidingluconat | | 0,01 | Gew.-% |
| Hyaluronsäure-NaSalz | | 0,03 | Gew.-% |
| Ethanol (90%) | | 25,0 | Gew.-% |
| Pfefferminzöl | | 0,29 | Gew.-% |
| Aqua purificata | ad | 100 | Gew.-% |

werden getrennt eingewogen. Anschließend wird Dexamethasonacetat in Ethanol gelöst. Hyaluronsäurenatriumsalz und Chlorhexidindigluconat werden in einem Teil des gereinigten Wassers gelöst. In der so erhaltenen Mischung wird Silbereiweisacetyltannat kolloidal gelöst. Die Dexamethasonacetat/Ethanol-Lösung wird hinzugefügt und in das erhaltene Gemisch Pfefferminzöl eingetropft. Anschließend wird mit gereinigtem Wasser auf 100 Gew.-% ergänzt. Die daraus resultierende pharmazeutische Zubereitung wird in eine Sprayflasche mit Zerstäuber abgefüllt.

### Beispiel 2

Sechs Patienten im Alter zwischen 18 und 57 Jahren ( 18, 23, 29, 41, 43, 57 ) mit Beschwerden zeigten als Hauptsymptom Heiserkeit. Vier dieser Patienten klagten darüber hinaus noch über Halsschmerzen. Drei weitere Patienten litten zusätzlich unter Reizhusten.

Über einen Zeitraum von 4 bis 9 Tagen wurde die gemäß Beispiel 1 erzeugte Zubereitung als Sprühlösung appliziert. Nach zweitägiger Applikation kam es bei allen Patienten zu einer subjektiv deutlichen Besserung der Heiserkeit sowie der Halsschmerzen. Der Reizhusten besserte sich bei einem Patienten erst nach 7 Tagen.

Die Patienten wurden nach dem ersten Untersuchungstag an Tag 3, 5 und an Tag 10 wieder einbestellt. Das klinische Hauptkriterium waren gerötete, geschwollene, symmetrisch bewegliche Stimmbänder. Des Weiteren eine gerötete Schleimhaut in Hypopharynx und Larynx.

Ausschlusskriterien waren sichtbare eitrige Beläge der Stimmbänder oder im Bereich der oberen Atemwege, eine bekannte Allergie gegen ein Bestandteil der Lösung oder ein bekannter Diabetes mellitus.

Bei allen Patienten war nach 3 Tagen ein deutlicher Rückgang der Stimmbandschwellung und Rötung zu verzeichnen, bei 4 von 6 war nach 5 Tagen eine weitest gehende Ausheilung zu beobachten.

## Patentansprüche

1. Pharmazeutische Zubereitung zur topisch-oralen Anwendung auf Schleimhäuten im Kehlkopf- und/oder Rachenraum, **dadurch gekennzeichnet, dass** sie mindestens die Komponenten
a) 0,01 bis 0,7 Gew.-% eines Kortikoids,
b) 0,01 bis 5 Gew.-% einer Metallverbindung,
c) 0,01 bis 5 Gew.-% eines Adstringens,
d) 0,005 bis 0,03 Gew.-% eines Desinfektionsmittels und
e) 0,01 bis 0,06 Gew.-% Hyaluronsäure/Hyaluronsäuresalz,
umfasst, worin die Zubereitung mit einem pharmazeutisch verträglichen Lösungsmittel zu 100 Gew.-% ergänzt wird, zur Verwendung in der symptomatischen Therapie einer viralen Laryngitis.

2. Zubereitung zur Verwendung nach Anspruch 1, **dadurch gekennzeichnet, dass** sie zusätzlich als Komponente
f) 0, 01 bis 0,4 Gew.-% eines ätherischen Öls
umfasst.

3. Zubereitung zur Verwendung nach Anspruch 1 oder 2, **dadurch gekennzeichnet, dass** sie zusätzlich als Komponente
g) 10,0 bis 40,0 Gew.-% Ethanol
umfasst.

4. Zubereitung zur Verwendung nach Anspruch 1 oder 2, **dadurch gekennzeichnet, dass** das Kortikoid ausgewählt ist aus Dexamethason, Hydrocortison, Prednisolon, Clobetason, Flumetason, Fluocortin, Fluperolon, Fluorometholon, Flupredniden, Desonid, Triamcinolon, Alclometason, Hydrocortisonbuteprat, Clocortolon, Betamethason, Fluclorolon, Desoximetason, Fluocinolonacetonid, Fluocortolon, Diflucortolon, Fludroxycortid, Fluocinonid, Budesonid ,Diflorason, Amcinonid, Halometason, Methylprednisolonaceponat, Beclometason, Hydrocortisonaceponat, Fluticason, Prednicarbat, Difluprednat, Ulobetasol, Clobetasol, Halcinonid, deren pharmazeutisch verträglichen Salze und Kombinationen davon.

5. Zubereitung zur Verwendung nach einem der voranstehenden Ansprüche, **dadurch gekennzeichnet, dass** die Metallverbindung ausgewählt ist aus Mn, Ag, Zn, Sn, Fe, Cu, Al, Ti, Kombinationen und pharmazeutisch verträglichen Salzen oder Oxidverbindungen davon.

6. Zubereitung zur Verwendung nach einem der voranstehenden Ansprüche **dadurch gekennzeichnet, dass** die Metallverbindung eine Metall-Eiweißverbindung ist, bevorzugt eine Silber-Eiweißverbindung.

7. Zubereitung zur Verwendung nach einem der voranstehenden Ansprüche, **dadurch gekennzeichnet, dass** das Adstringens ausgewählt ist aus Catechingerbstoffen, polymeren Proanthocyanidinen, oligomeren Proanthocyanidinen, Oligomeren oder Polymeren der Catechine, Catechin, Epicatechin, Flavonoiden, hydrolysierbaren Gerbstoffen, Gallotanninen, Estern der Gallussäure oder Derivaten davon, Tanninen, Kaffeesäure- und Phloroglucinderivaten, Labiatengerbstoffen, Rosmarinsäure oder Derivaten davon, Alaun, Tonerde und Kombinationen davon.

8. Zubereitung zur Verwendung nach einem der voranstehenden Ansprüche, **dadurch gekennzeichnet, dass** das Desinfektionsmittel nach Anspruch 1 ausgewählt ist aus Chlorhexidin-Digluconat, Hexetidin, Ethanol, Propanol, Dichlorbenzylalkohol, Amylmetakresol, Menthol, Levomenthol, Dequaliniumchlorid, Iod, Povidon-Iod, Gentianaviolett und Kombinationen davon.

9. Zubereitung zur Verwendung nach einem der voranstehenden Ansprüche, **dadurch gekennzeichnet, dass** das ätherische Öl ausgewählt ist aus Orange, Kamille, Schafgarbe, Salbei, Pfefferminze und Kombinationen davon.

10. Zubereitung zur Verwendung nach einem der voranstehenden Ansprüche, **dadurch gekennzeichnet, dass** das Lösungsmittel Wasser und/oder Ethanol ist.

11. Zubereitung zur Verwendung nach einem der voranstehenden Ansprüche, **dadurch gekennzeichnet, dass** sie einen oder mehrere Zusatzstoffe enthält, ausgewählt aus pharmazeutischen Hilfsstoffen, galenischen Hilfsstoffen, Zuckeraustauschstoffen, Zuckerersatzstoffen, Säuerungsmitteln, Lösungsvermittlern, Verdickungsmitteln, Füllmitteln, Farbstoffen, Konservierungsmitteln, Lokalanästhetika, Aromen, Geschmacksstoffen, Provitaminen, Vitaminen und Kombinationen davon.

12. Zubereitung zur Verwendung nach einem der voranstehenden Ansprüche, **dadurch gekennzeichnet, dass** die Zubereitung ein Spray zur Anwendung im Rachenraum ist.

13. Zubereitung zur Verwendung nach einem der Ansprüche 1 bis 12 zur Behandlung einer viralen Laryngitis.

## Claims

1. Pharmaceutical preparation for topical oral use on mucous membranes in the laryngeal and/or pharyngeal cavity, **characterized in that** it comprises at least the components
a) 0.01 to 0.7 wt.% of a corticosteroid,
b) 0.01 to 5 wt.% of a metal compound,
c) 0.01 to 5 wt.% of an astringent,
d) 0.005 to 0.03 wt.% of a disinfectant, and
e) 0.01 to 0.06 wt.% hyaluronic acid/hyaluronic acid salt,
the preparation being supplemented to 100 wt.% with a pharmaceutically acceptable solvent for use in symptomatic therapy of viral laryngitis.

2. Preparation for use according to claim 1, **characterized in that** it additionally comprises
f) 0.01 to 0.4 wt.% of an essential oil
as a component.

3. Preparation for use according to either claim 1 or claim 2, **characterized in that** it additionally comprises
g) 10.0 to 40.0 wt.% ethanol
as a component.

4. Preparation for use according to either claim 1 or claim 2, **characterized in that** the corticosteroid is selected from dexamethasone, hydrocortisone, prednisolone, clobetasone, flumetasone, fluocortin, fluperolone, fluorometholone, fluprednidene, desonide, triamcinolone, alclometasone, hydrocortisone buteprate, clocortolone, betamethasone, fluclorolone, desoximetasone, fluocinolone acetonide, fluocortolone, diflucortolone, fludroxycortide, fluocinonide, budesonide, diflorasone, amcinonide, halometasone, methylprednisolone aceponate, beclometasone, hydrocortisone aceponate, fluticasone, prednicarbate, difluprednate, ulobetasol, clobetasol, halcinonide, the pharmaceutically acceptable salts thereof, and combinations thereof.

5. Preparation for use according to any of the preceding claims,
**characterized in that** the metal compound is selected from Mn, Ag, Zn, Sn, Fe, Cu, Al, Ti, combinations and pharmaceutically acceptable salts thereof, or oxide compounds thereof.

6. Preparation for use according to any of the preceding claims
**characterized in that** the metal compound is a metal protein compound, preferably a silver protein compound.

7. Preparation for use according to any of the preceding claims,
**characterized in that** the astringent is selected from catechin tannins, polymeric proanthocyanidins, oligomeric proanthocyanidins, oligomers or polymers of catechins, catechin, epicatechin, flavonoids, hydrolyzable tannins, gallotannins, esters of gallic acid or derivatives thereof, tannins, caffeic acid derivatives and phloroglucinol derivatives, Lamiacae tannins, rosemarinic acid or derivatives thereof, alum, alumina and combinations thereof.

8. Preparation for use according to any of the preceding claims,
**characterized in that** the disinfectant according to claim 1 is selected from chlorhexidine digluconate, hexetidine, ethanol, propanol, dichlorobenzyl alcohol, amylmetacresol, menthol, levomenthol, dequalinium chloride, iodine, povidone iodine, gentian violet and combinations thereof.

9. Preparation for use according to any of the preceding claims,
**characterized in that** the essential oil is selected from orange, chamomile, yarrow, sage, peppermint and combinations thereof.

10. Preparation for use according to any of the preceding claims,
**characterized in that** the solvent is water and/or ethanol.

11. Preparation for use according to any of the preceding claims,
**characterized in that** it contains one or more additives selected from pharmaceutical excipients, galenic excipients, sugar substitutes, sweeteners, acidifiers, solubilizers, thickeners, fillers, dyes, preservatives, local anesthetics, fragrances, flavorings, provitamins, vitamins, and combinations thereof.

12. Preparation for use according to any of the preceding claims,
**characterized in that** the preparation is a spray for use in the pharyngeal cavity.

13. Preparation for use according to any of claims 1 to 12 for treating a viral laryngitis.

## Revendications

1. Préparation pharmaceutique destinée à être appliquée par voie topique/orale sur des muqueuses dans le larynx et/ou le pharynx, **caractérisée en ce qu'**elle comprend au moins les composants suivants
a) 0,01 à 0,7 % en poids d'un corticoïde,
b) 0,01 à 5 % en poids d'un composé métallique,
c) 0,01 à 5 % en poids d'un astringent,
d) 0,005 à 0,03 % en poids d'un désinfectant et
e) 0,01 à 0,06 % en poids d'acide hyaluronique/sel d'acide hyaluronique,
la préparation étant complétée à 100 % en poids par un solvant pharmaceutiquement acceptable, laquelle préparation étant destinée à être utilisée dans le traitement symptomatique d'une laryngite virale.

2. Préparation destinée à être utilisée selon la revendication 1,
**caractérisée en ce qu'**elle comprend en outre, comme composant
f) 0, 01 à 0,4 % en poids d'une huile essentielle.

3. Préparation destinée à être utilisée selon la revendication 1 ou 2, **caractérisée en ce qu'**elle comprend en outre, comme composant
g) 10,0 à 40,0 % en poids d'éthanol.

4. Préparation destinée à être utilisée selon la revendication 1 ou 2, **caractérisée en ce que** le corticoïde est choisi parmi la dexaméthasone, hydrocortisone, prednisolone, clobétasone, flumétasone, fluocortine, flupérolone, fluorométholone, fluprednidène, désonide, triamcinolone, alclométasone, butéprate d'hydrocortisone, clocortolone, bétamethasone, fluclorolone, désoximétasone, acétonide de fluocinolone, fluocortolone, diflucortolone, fludroxycortide, fluocinonide, budésonide, diflorasone, amcinonide, halométasone, acéponate de méthylprednisolone, béclométasone, acéponate d'hydrocortisone, fluticasone, prednicarbate, difluprednate, ulobétasol, clobétasol, halcinonide, leurs sels pharmaceutiquement acceptables et leurs combinaisons.

5. Préparation destinée à être utilisée selon l'une des revendications précédentes, **caractérisée en ce que** le composé métallique est choisi parmi Mn, Ag, Zn, Sn, Fe, Cu, Al, Ti, des combinaisons et des sels ou composés oxydes pharmaceutiquement acceptables de ceux-ci.

6. Préparation destinée à être utilisée selon l'une des revendications précédentes, **caractérisée en ce que** le composé métallique est un composé métal/protéine, de préférence un composé argent/protéine.

7. Préparation destinée à être utilisée selon l'une des revendications précédentes, **caractérisée en ce que** l'astringent est choisi parmi les tannins de catéchine, proanthocyanidines polymères, proanthocyanidines oligomères, oligomères ou polymères des catéchines, catéchine, épicatéchine, flavonoïdes, tannins hydrolysables, gallotannins, esters de l'acide gallique ou leurs dérivés, tannins, dérivés de l'acide caféique et de la phloroglucine, tannins de Labiées, acide rosmarinique ou ses dérivés, alun, argile et des combinaisons de ceux-ci.

8. Préparation destinée à être utilisée selon l'une des revendications précédentes, **caractérisée en ce que** le désinfectant selon la revendication 1 est choisi parmi le digluconate de chlorhexidine, hexétidine, éthanol, propanol, alcool dichlorobenzylique, amylmétacrésol, menthol, lévomenthol, chlorure de déqualinium, iode, povidone iodée, violet de gentiane et des combinaisons de ceux-ci.

9. Préparation destinée à être utilisée selon l'une des revendications précédentes, **caractérisée en ce que** l'huile essentielle est choisie parmi l'orange, camomille, achillée millefeuille, sauge, menthe poivrée et des combinaisons de celles-ci.

10. Préparation destinée à être utilisée selon l'une des revendications précédentes, **caractérisée en ce que** le solvant est l'eau et/ou l'éthanol.

11. Préparation destinée à être utilisée selon l'une des revendications précédentes, **caractérisée en ce qu'**elle contient un ou plusieurs additifs choisis parmi les adjuvants pharmaceutiques, adjuvants galéniques, substituts de sucre, édulcorants, acidifiants, promoteurs de solubilisation, épaississants, charges, colorants, conservateurs, anesthésiques locaux, arômes, aromatisants, provitamines, vitamines et des combinaisons de ceux-ci.

12. Préparation destinée à être utilisée selon l'une des revendications précédentes, **caractérisée en ce que** la préparation est un spray destiné à être appliqué dans le pharynx.

13. Préparation destinée à être utilisée selon l'une des revendications 1 à 12 pour le traitement d'une laryngite virale.
